# EUROPEAN PATENT SPECIFICATION

(11) **EP 1 183 361 B1**
(45) Date of publication and mention of the grant of the patent: **30.03.2005**
(21) Application number: 00936206.2
(22) Date of filing: 23.05.2000
(51) Int. Cl.: C12N 15/24, C12N 15/86, A61K 48/00, A61K 35/76, A61P 29/00

(54) **GENE THERAPY OF PULMONARY DISEASE**
GENTHERAPY VON LUNGENKRANKHEITEN
THERAPIE GENIQUE POUR MALADIE PULMONAIRE

(30) Priority: 25.05.1999 US 318077
(43) Date of publication of application: 06.03.2002
(73) Proprietor: CANJI, Inc., San Diego, CA 92121 (US)
(72) Inventor: LAFACE, Drake, M., San Diego, CA 92126 (US); MINTER, Rebecca, M., Gainesville, FL 32606 (US); MOLDAWER, Lyle, L., Gainesville, FL 32605 (US)
(74) Representative: UEXKÜLL & STOLBERG
(86) International application number: PCT/US2000/014146
(87) International publication number: WO 2000/071718

(56) References cited:
- EP-A- 1 013 764
- WO-A-98/51807
- NAST CYNTHIA C ET AL: "Long-term allograft acceptance in a patient with posttransplant lymphoproliferative disorder: Correlation with intragraft viral interleukin-10." TRANSPLANTATION (BALTIMORE), vol. 64, no. 11, 15 December 1997 (1997-12-15), pages 1578-1582, XP000918351 ISSN: 0041-1337
- WHALEN, JD ET AL: "Adenoviral transfer of the viral IL-10 gene periarticularly to mouse paws suppresses development of collagen-induced arthritis in both injected and uninjected paws" J IMMUNOL, vol. 162, no. 6, 15 March 1999 (1999-03-15), pages 3625-3632, XP002149335
- BOEHLER A ET AL: "Adenovirus-mediated interleukin-10 gene transfer inhibits post-transplant fibrous airway obliteration in an animal model of bronchiolitis obliterans." HUMAN GENE THERAPY, vol. 9, no. 4, 1 March 1998 (1998-03-01), pages 541-551, XP000918371 ISSN: 1043-0342

## Description

### Background of the Invention

Immune responses are mediated in large part by cells derived from the hematopoietic lineage. Regulation of immune cells in inflammatory responses is mediated both by direct cell-cell interactions and indirectly by secretion of cytokines and chemokines which elicit various phenotypic responses upon receptor ligation. Innate cytokine responses play a fundamental role in initiating inflammation and afferent immune responses and cytokines also regulate the type of efferent immune response by various immune cells including, but not limited to, T-cells, B-cells, NK cells, macrophages, dendritic cells (and other antigen presenting cells), neutrophils and other inflammatory cells. An imbalance of cytokine responses can result in the induction or exacerbation of destructive inflammatory responses resulting in a pathological condition in the host. Moreover, cytokine mediated inflammatory responses can greatly limit the effectiveness of gene therapy procedures by initiating and upregulating immune responses to transduced cells and to the therapeutic transgene. These processes are mediated in large part by pro-inflammatory cytokines such as IL-1-α, IL-1-β, IL-6, IL-8 and TNF-α. Therefore, methods are needed to modulate cytokine mediated inflammatory responses for the treatment of pathological inflammatory responses and to modulate immune responses which prevent effective gene based therapeutics.

A severe form of auto-destructive inflammation with little effective treatment intervention choice is Adult Respiratory Distress Syndrome (also referred to as Acute Respiratory Distress Syndrome and hereinafter collectively referred to as ARDS). ARDS was first described in 1967 (Ashbaugh, *et al*. (1967) Lancet 2:319-323.) and is characterized by diffuse pulmonary microvascular injury resulting in increased permeability and hypoxemiea caused by intrapulmonary shunts. The following criteria are used to define ARDS (Dal Nogare (1989) Am. J. Med. Sci. 298 (6): 413-430, Murray *et a*l. (1988) Am. Rev. Respir. Dis. 138:720-723)
1) clinical evidence of respiratory distress.
2) hypoxemia that is difficult to correct with oxygen supplementation.
3) hemodynamic evidence of pulmonary artery occlusion (wedge) pressure <18 mm Hg.
4) thoracic static compliance less than 40 mL/cm of water.
5) chest radiograph revealing diffuse bilateral airspace disease; pulmonary edema.
Although diagnosis of ARDS is complex and to a large degree based on exclusion, there are a set of physiologic criteria that can be employed for diagnosis. During the initial clinical phase of ARDS patients generally present with dyspnea (labored respiration) and tachypnea (increased rate of respiration), pulmonary hypertension, and hyperventillation induced alkalosis. Progression to the second phase usually occurs with in 12-24 hours and testing of arterial blood gases usually reveals severe hypoxemia (e.g., PaO2 < 200 mm Hg and as low as 50mm Hg). Moreover, the hypoxemia with ARDS is difficult to correct with supplemental oxygen ventilation support therapy. In addition radiographic analysis reveals diffuse pulmonary consolidations indicative of pulmonary edema and fluffy infiltrate opacities as polymorphonuclear leukocyte (PMN) infiltration progresses.

The first two stages of ARDS progression (i.e., 12-72 hour after onset) is a critical window for intervention as the syndrome can be reversed if the initiating factors and the inflammatory mediators can be controlled. An early diagnosis may also be facilitated if the initiating stimulus is known as in determination of sepsis, aspiration of gastric contents, multiple transfusions, severe fractures, bums, pancreatitis or severe trauma. Upon progression to a third stage of ARDS pulmonary hypertension increases, heart rates increases to compensate for hypoxemia and mechanical ventilation supportive therapy is generally required. Pathologically the cellular infiltrates are more dense with continued neutrophil infiltration and increasing monuclear, lymphocyte and fibroblast cell infiltrates. This is associated with an increase in mortality of greater than 50%. However, successful intervention can still result in complete resolution if the disease process is stopped. Progression to the fourth phase in which pulmonary fibrosis and progressive respiratory failure occurs is generally associated with a poor prognosis and a mortality of greater than 80% following progression to multiple organ system failure (MOSF).

Based on the progressive pathological process of ARDS and the associated rise in mortality rates, early intervention with treatments which can modulate the inflammatory processes that mediate ARDS may be essential. Thus, functional tests and monitoring of the pulmonary function is essential and should be an integral part of early diagnosis and utilized to determine the best time for medical intervention with newly developed treatments. A pulmonary artery catheter should be placed in patients with suspected ARDS to serve as a diagnostic tool and to monitor progress following treatment.

Despite extensive investigation to elucidate ARDS pathology, little therapeutic progress has been made and supportive care remains the predominant treatment regimen. There are approximately 150,000 cases of ARDS per year in the United States and a relatively high mortality rate of nearly 50% (ranging from 30%-70%). The pathological process of ARDS is essentially a form of lung inflammation. A number of conditions predispose patients for the development of ARDS including major trauma, sepsis, pancreatitis, hemorragic shock aspiration of gastric contents, pneumonia, pulmonary contusion, burns, smoke inhalation and multiple blood transfusions. Moreover, unresolved progressive ARDS often results in the development of multisystem organ failure syndrome (MOSF) which invariably is associated with a very grave prognosis.

While the conditions predisposing patients for ARDS are diverse, a common component is polymorphonuclear leukocyte (PMN) infiltration and the induction of cytokine responses associated with the onset of ARDS. The mere presence of large numbers of PMNs is not injurious to the normal lung but rather requires activation as an initial step to mediate pulmonary injury. Stimuli which can activate PMNs include bacterial products such as lipopolysaccharide (LPS) and formyl-methionine-leucine-phenylalanine (FMLP), which can stimulate production of large amounts of TNF-α by macrophages. Hence cytokine responses to various inflammatory stimuli can initiate pulmonary injury. Additional mediators of lung injury in ARDS include free radicals, reactive oxygen intermediates, arachidonic acid metabolites and proteases. Since ventilation support therapies do not significantly reduce mortality, methods are needed to modulate the inflammatory response itself. Moreover, since multiple mediators of the alveolar capillary barrier disruption are involved, combination therapies which rectify the cytokine imbalances as well as the free radicals, arachidonic acid metabolites and proteases may be required.

The pleiotropic cytokine, IL-10 is a potent regulator of cytokine synthesis and suppressor of effector functions of macrophages, T cells and NK cells. IL-10 has been identified as useful in the treatment of many indications including adoptive immunotherapy of cancer (United States Patent No. 5,776,451 issued January 16, 1991), septic shock (United States Patent No 5,833,976 issued November 10, 1998 and United States Patent 5,753,218 issued May 19, 1998), to modulate inflammation or T-cell mediated immune function (United States Patent No. 5,837,293 issued November 17, 1998), inflammatory bowel disease (United States Patent No. 5,368,854 issued October 29, 1994), in combination with IL-1 to treat delayed hypersensitivity (United States Patent No. 5,601,815 issued February 11, 1997), insulin dependent diabetes mellitus (United States Patent No. 5,827, 513 issued October 27, 1998), cancer in combination with IL-2 and IFN-γ (United States Patent No. 5,871,725 issued February 16, 1999), acute leukemia (United States Patent No. 5,770,190 issued June 23, 1998), as a vaccine adjuvant (United States Patent No. 5,866,134 issued March 24, 1995).

An important element of IL-10 activity is that the predominant functional phenotypes induced by IL-10 depends on the local context in which the cytokine is produced and bound by its receptor. Specifically, IL-10 inhibits cytokine synthesis, including IFN-γ, TNF-α, GM-CSF and lymphotoxin, by human peripheral blood mononuclear cells (PBMC) both at the protein and RNA levels (Vieira *et al*. (1991) Proc. Natl. Acad. Sci. USA 88:1172-1176, Hsu, et al. (1990) Science 250:830-832). Moreover, IL-10 can inhibit T-cell proliferation. The inhibitory functions are mediated in an antigen presenting cell-dependent (APC-dependent) manner. The mechanism by which IL-10 inhibits APC-dependent functions is not completely understood but is associated with downregulation of constituitive and IFNγ-induced expression of MHC class II molecules as well as the production of cytokines by macrophages and monocytes. Moreover, LPS and IFN-γ activation induced production of key pro-inflammatory monokines and growth factors by monocytes and macrophages, including EL-1-α, IL-1-β IL-6, EL-8, TNF-α, GM-CSF and G-CSF, has been reported to be strongly inhibited by IL-10. These anti-inflammatory capacities have been demonstrated *in vitro* as well as in *vivo* in an LPS-induced shock pre-clinical animal model (Fiorentino *et al*. (1991) J. Immunol. 147:3815-3822, O'Garra, *et al*. (1992) Int. Rev. Immunol 8:219-234). This anti-inflammatory activity is quite pertinent as LPS-induced inflammation share numerous aspects with the various stimuli that induce ARDS.

Pertinent to gene therapy delivery of IL-10 expression, endogenously produced IL-10 inhibits the production of monocytes in an autoregulatory manner. This may be particularly important following transduction of monocytes, macrophages , dendritic cells or other antigen presenting cells (APC) and may influence the local environment in which IL-10 is expressed as well as the capacity to promote anti-inflammatory activities in gene therapy scenarios and for localized treatment of inflammatory pulmonary disease such as ARDS. Another set of important inflammatory mediators, which are associated with the development of ARDS, which are inhibited by IL-10 are reactive oxygen intermediates (H₂O₂) and reactive nitrogen intermediates (NO) following activation of macrophages. This inhibition has been demonstrated to be due to TNF-α inhibition (Bogdan *et al*. (1992) J. Biol. Chem. Nov 15;267(32):23301-8).

As noted above, human IL-10 is a pleiotropic cytokine. In addition to the numerous anti-inflammatory activities described above, IL-10 also has been shown to augment several immunological functions. For example human IL-10 (hIL-10) has been shown to be costimulatory for B-cell proliferation and immunoglobulin production (particularly in combination with CD40 crosslinking). In addition, human IL-10 has been shown to be a chemotactic factor for CD8+ T-cells and to promote CD8+ cell proliferation. Viral IL-10 (vIL-10) derived from the EBV BCRF1 gene has biological responses that differ significantly in some contexts from the actions of human IL-10. While retaining the capacity to suppress anti-inflammatory responses, vIL-10 demonstrates a substantially reduced augmentation of immune functions such as the capacity to promote costimulatory functions. This makes vIL-10 an attractive candidate for IL-10 gene therapy of inflammatory diseases were anti-inflammatory responses in the relative absence of immune augmentation may be desirable. Although ARDS is a primary disease target for novel IL-10 therapies, other chronic and acute inflammatory diseases are amenable to treatment by this therapy.

### Summary of the Invention

The present invention provides a method of treating an individual suffering from a pulmonary inflammatory disease by the administration of recombinant gene therapy vectors expressing viral IL-10 and provides a method for the use of recombinant vectors expressing viral IL-10 to facilitate multi-dose gene therapy treatment regimens.

### Brief Description of the Figures

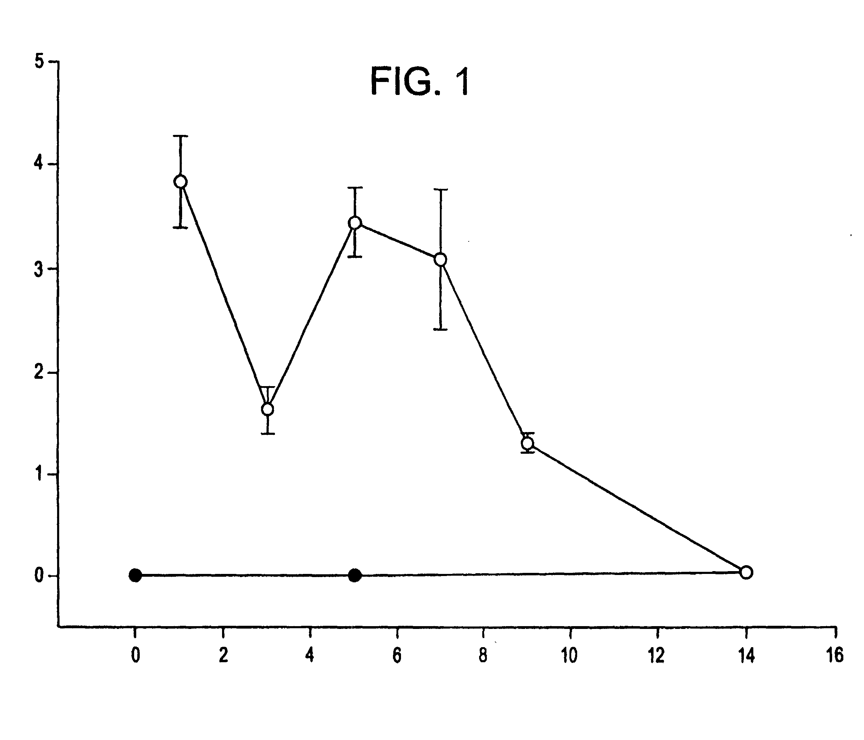

Figure 1 is a graphical representation of β-galactosidase levels (○) following intratracheal administration of rAd/β-gal versus time. Administration of buffer alone was used as a control (•). The vertical axis is a measure of β-galactosidase levels in micrograms of β- galactosidase per gram of wet lung weight homogenate as determined in accordance with the teaching of Example 5. The horizontal axis is the time following administration measured in days.

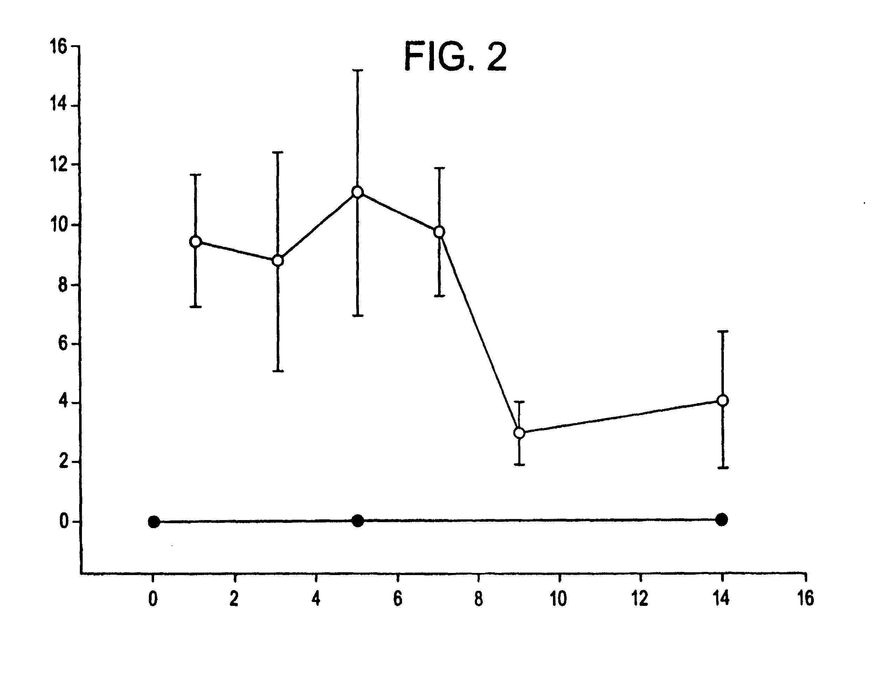

Figure 2 is a graphical representation of hIL-10 (○) lung levels following intratracheal administration of rAd/hIL-10 versus time. Administration of buffer alone was used as a control (•). The vertical axis represents hIL-10 levels in nanograms of hIL-10 protein per gram of wet lung weight as determined in accordance with the teaching of Example 6. The horizontal axis is the time following administration measured in days. Lung levels of hIL-10 in mice treated with rAd/hIL-10 are represented by the open circle (○). Lung levels of human IL-10 in the lungs of mice treated with buffer are represented by the closed circle (•), and were undetectable. The differences in the magnitude of expression between the two experimental groups are statistically significant.

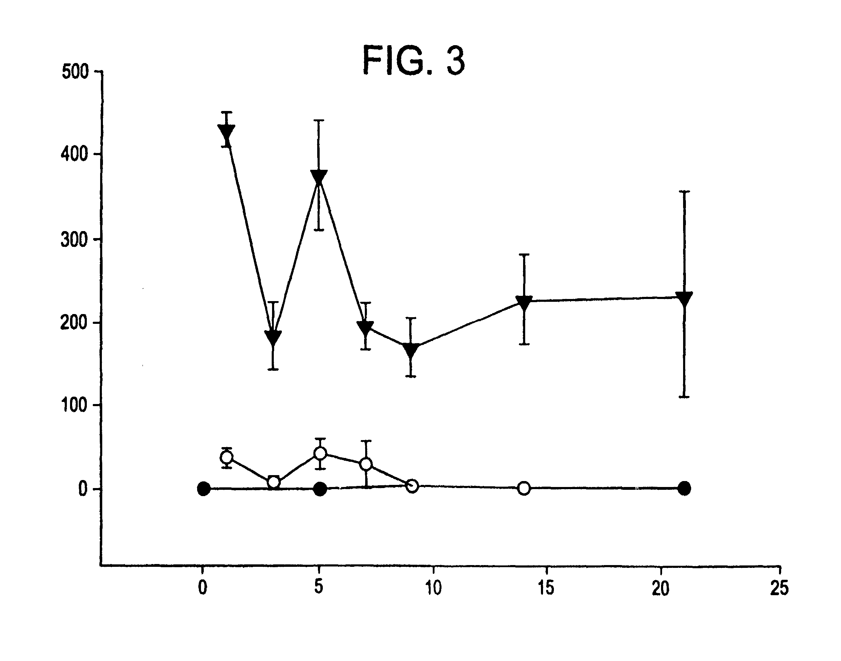

Figure 3 is a graphical representation of vIL-10 and hIL-10 levels in the lung following intratracheal administration of rAd/vIL-10 and rAd/hIL-10 versus time. The vertical axis represents of IL-10 levels in nanograms of IL-10 protein per gram of wet weight lung homogenate as determined in accordance with the teaching of Example 6. The horizontal axis the time following administration measured in days. Drug levels of vIL-10 in mice treated with rAd/vIL-10 are reported by the closed triangle (▼). Drug levels of human IL-10 in mice treated with rAd/hIL10 are represented by the open circle (○). Lung levles of human and viral IL-10 in mice treated with buffer are represented by the closed circle (•) and were undetectable.

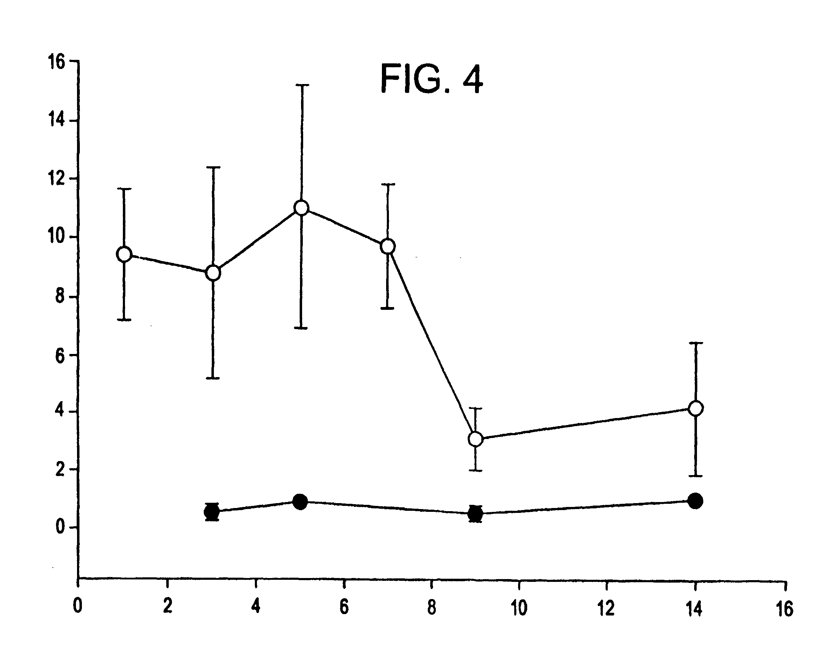

Figure 4 is a graphical representation comparing hIL-10 levels in serum and lung tissues following intratracheal administration of rAd/hIL-10 versus time. The vertical axis represents hIL-10 levels in nanograms of hIL-10 protein per gram of tissue (lung) or ml of plasma (plasma) as determined in accordance with the teaching of Example 6. The horizontal axis the time following administration measured in days. Lung levels of hIL-10 in mice treated with rAd/hIL-10 are represented by the open circle (○). Plasma levels of hIL-10 in mice treated with rAd/hIL-10 are represented by the closed circle (•).

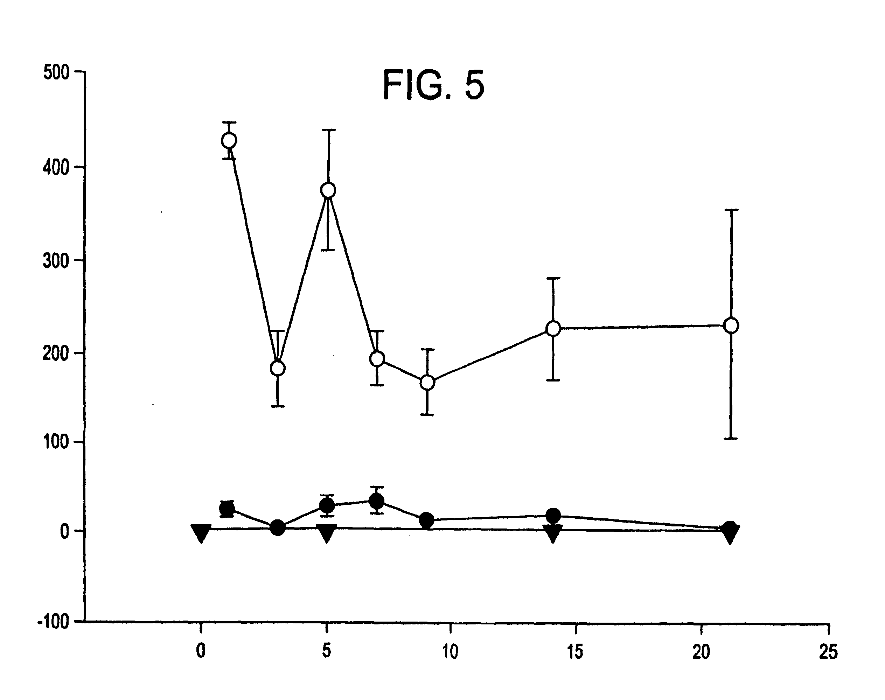

Figure 5 is a graphical representation comparing vIL-10 levels in serum and lung tissues following intratracheal administration of either rAd/vIL-10 or only buffer versus time. The vertical axis represents vIL-10 levels in nanograms of vIL-10 protein per gram of tissue (lung) or ml of plasma (plasma) as determined in accordance with the teaching of Example 6. The horizontal axis the time following administration measured in days. Lung levels of vIL-10 in mice treated with rAd/vIL-10 are represented by the open circle (○). Plasma levels of vIL-10 in mice treated with rAd/vIL-10 are represented by the closed circle (•). Lung levels of viral IL-10 in the lungs of mice treated with buffer are represented by the closed triangle ( ▲).

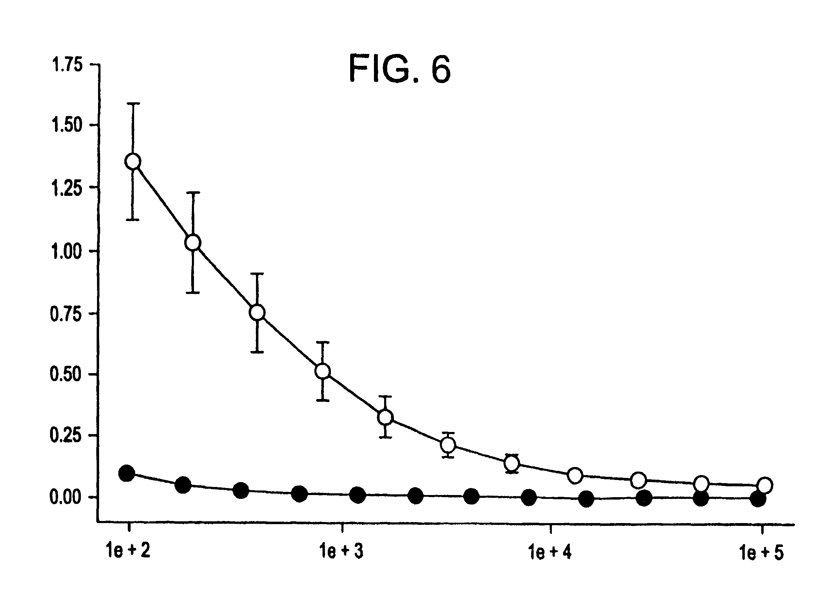

Figure 6 is a graphical representation illustrating anti-hIL-10 and anti-β-galactosidase serum antibody levels following intratracheal administration of rAd/β-gal and rAd/hIL-10. The vertical axis represents absorbance units (quantity of antibody) and the horizontal axis represents the serum dilution factor. hIL-10 is represented by the solid circle (•) and β-galactosidase by the open circle (○). The differences in antibody response were highly significant.

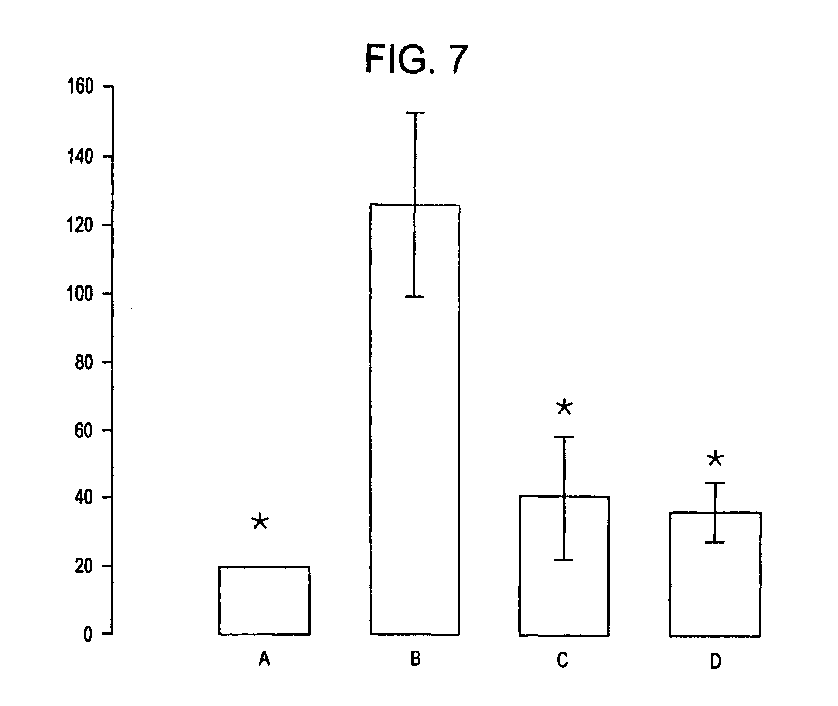

Figure 7 is a histogram representing anti-adenoviral antibody titers following administration of buffer (Column A), rAd/β-gal (Column B), rAd/hIL-10, (Column C) and rAd/vIL-10 (Column D) by intratracheal administration of 1x10¹⁰ particles of virus. Antibody titers were determined at 14 days after instillation. the y-column indicates antibody titer, inverse dilution for EC₅₀. Asterists indicate that Groups A, C, and D were statistically different from Group B. Groups C and D were not different from Group A.

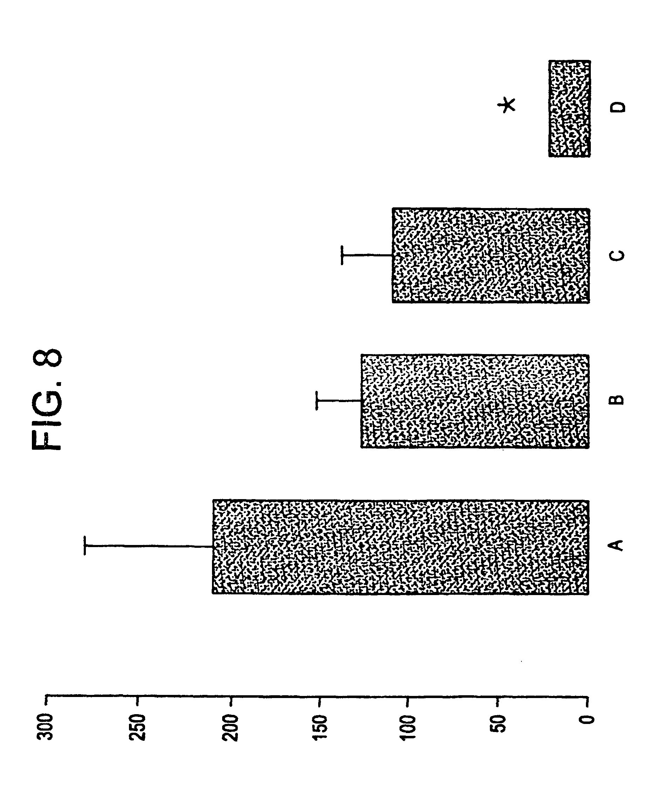

Figure 8 is a histogram representing anti-adenoviral antibody titers following administration of a combination of 5x10⁹ rAd/β-gal and 5x10⁹ rAd/empty cassette (Column A), 5x10⁹ rAd/hIL-10 and 5x10⁹ rAd/β-gal (Column B), 5x10⁹ rAd/vIL-10 and 5x10⁹ rAd/β-gal (Column C), and 5x10⁹ rAd/vIL-10 and 5x10⁹ rAd/empty cassette (Column D) particles of each virus. Antibody titers were determined at 14 days after instillation. The y-column indicates the antibody titer, inverse dilution for EC₅₀. The asterisk indicates that group D was significantly different from Groups A and B, but not C.

### Detailed Description of the Invention

A study was performed to evaluate the efficacy of recombinant adenoviral vectors expressing either β-galactosidase, human IL-10 or viral IL-10 to reduce the magnitude of pulmonary inflammation associated with adenoviral instillation and to extend transgene expression. Pre-clinical experiments were performed in a mouse model of pulmonary inflammation created by the administration of recombinant adenoviral expression vectors. It is understood that although the inflammation produced by adenoviral instillation at these doses is mild, the underlying mechanisms (i.e. cytokine mediated) are presumed to be similar to the mechanims of lung injury associated with other chronic and acute inflammatory processes, such as ARDS and pneumonia. Administration of rAd vectors expressing β-galactosidase (rAd/β-gal) intratracheally in C57BL/6 mice were used to induce a biphasic inflammatory response, characterized by an early neutrophil and macrophage infiltration, followed by lymphocyte infiltration and apoptosis. Moreover, there is an immediate innate pro-inflammatory cytokine response to rAd/β-gal instillation in the lung that occurs over the first 24 hours and then a secondary peak of TNF-α responses over the next 5-7 days. This inflammatory response is associated with mild parenchymal injury and a decrease in the duration of transgene expression. Consequently this model provided a basis for studying the effects of IL-10 gene therapy to suppress the pro-inflammatory mediators associated with the development of lung injury. As more fully described in the Example 4 herein, C57BL/6 female mice received intra-tracheal instillation of 1x10¹⁰ particles (PN/mouse) of recombinant adenovirus (rAd) expressing human IL-10 (hIL-10), viral IL-10 (vIL-10) or β-galactosidase (β-gal) prepared in accordance with the teaching of Examples 1, 2 and 3. Mice were sacrificed on days 1, 3, 5, 7, 9 and 14 and assessed for cytokine responses (TNF-α, murine IL-6, IL-1 and myeloperoxidase levels) and for transgene expression (hIL-10, vIL10 and β-gal) in lung in accordance with the teaching of the Examples 5 and 6.

The results of these experiments are presented in graphical form in Figures 1, 2 and 3. As can be seen from the data presented in Figure 1, peak β-galactosidase expression levels were observed on days 1 and 5 and decreased to immeasurable levels by day 14. Similar results were obtained with rAd/hIL-10 in lung homogenates (presented in Figure 2). A greater than 95% reduction in β-gal and hIL-10 expression levels was observed by day 14 (p<0.001). In contrast as shown in Figure 3, administration of rAd/vIL-10 resulted in continued expression of vIL-10 at high levels (>150 ng/gm wet weight) in lung at all time points through day 14 (p<0.001 vs hIL-10). Viral IL-10 levels following lung instillation of rAd/vIL-10 are consistently five to ten times higher than hIL-10 levels (following rAd/hIL-10 administration) and has demonstrated persistent vIL-10 expression for up to 21 days.

vIL-10 and hIL-10 levels in the lung following intratracheal administration of rAd/vIL-10 and rAd/hIL-10 were compared to vIL-10 and hIL-10 levels in serum. Serum levels were determined by ELISA assay in substantial accordance with the teaching of Example 6. The results are presented in Figures 4 and 5 of the attached drawings. As can be seen from the data presented in Figure 4, serum levels of hIL-10 are persistently low while expression in lung demonstrates a short lived expression profile with peak levels at 11 nanograms/gram of wet lung). In contrast, the data presented in Figure 5 with vIL-10 vectors demonstrate a persistent high level of vIL-10 expression in the lung with low serum vIL-10 levels (similar to those obtained with hIL-10).

An additional experiment was performed in which rAd/hIL-10 and rAd/vIL-10 vectors were administered intravenously (at dosages of 1x10¹⁰, 5x10¹⁰ and 1x10¹¹)to BALB/c mice and the levels of hIL-10 and vIL-10 protein levels in serum determined on days 4, 7, 11 and 21 following administration in accordance with the teaching of Example 6. The results of these experiments demonstrated a short lived high expression level of both IL-10 species (peak on day 4) and a rapid decrease in expression on day 7 to an insignificant level on day 11.

These results demonstrate the unexpected advantage of direct administration of recombinant vectors encoding vIL-10 to the lung when compared to hIL-10. The consistently high levels of vIL-10 expression in lung tissue following rAd/vIL-10 administration are particularly unexpected in view of the transient and similar expression levels of hIL-10 and vIL-10 responses observed following intravenous administration of the same vectors. These results demonstrate that administration of recombinant vectors encoding vIL-10 to the lung results in much higher levels and persistent expression of IL-10 in lung tissue as compared to intravenous administration of human or viral IL-10 vectors.

An additional analysis of pro-inflammatory responses following intratracheal administration of rAd vectors expressing hIL-10, vIL-10 and β-galactosidase showed that both rAd/hIL10 and rAd/vIL10 treatments resulted in lower lung TNF-α production as compared to rAd/β-gal treated mice (0.48±0.18 and 0.63±0.10 vs. 1.5±0.4 ng/gm wet weight, p<0.05 at day one) suggesting that both hIL-10 and vIL-10 could suppress TNF-α cytokine responses. However, there were significant differences in the lung inflammatory response in mice treated with rAd/vIL-10 and rAdhIL-10. Mice treated with rAd/hIL-10 had a significantly higher neutrophil infiltration in the lung, as determined by myeloperoxidase (MPO), than mice treated with rAd/vIL-10 thoughout the 14 day study period (0.289 ± 0.016 versus 0.187 ± 0.016 U/gm wet weight; p<0.05), as determined by two-way analysis of variance. Although both rAd/vIL-10 and rAd/hIL-10 suppressed TNFα on day one, overall IL-1α concentrations (1.01 ± 0.15 versus 1.95 ± 0.15 ngs/gm wet weight; p<0.05) in the lung over the 14 days were significantly lower in rAd/vIL-10 treated mice than in rAd/hIL-10 treated mice, as determined by two-way analysis of variance. This latter finding was unexpected.

These results reflect the observed propensity of hIL-10 to induce pleiotropic responses including both anti-inflammatory and pro-inflammatory phenotypes as compared to vIL-10 which is associated primarily with suppression of the inflammatory responses to adenovirus instillation. Moreover, the results indicate that rAd gene transfer to the lung is associated with an inflammatory response characterized by increased TNF-α, MPO, and IL-1-α as well as, neutrophil cell infiltration. The results demonstrate that the administration of a recombinant vIL-10 expression vector produced a pronounced capacity to suppress the pro-inflammatory cytokine response that was unexpectedly different than from hIL-10 expression vector, demonstrated prolonged expression in the lungs, did not stimulate neutrophil infiltration in the lung to the extent seen with human IL-10 and had the capacity to down modulate cytokine responses induced by inflammatory stimuli in the lung.

The capacity of hIL-10 to suppress humoral responses to hIL-10 proteins following intratracheal administration rAd/hIL-10 was also evaluated. Serum was obtained from C57BL/6 mice 14 days following intratracheal administration of 1x10¹⁰ particles of rAdhIL-10 and rAd/β-gal vectors. The anti-IL-10 antibody response was evaluated by ELISA in accordance with the teaching of Example 7. The results are presented in Figure 6 of the accompanying drawings. These results demonstrate a dramatically lower transgene specific response following intratracheal administration of hIL-10 vector as compared to rAd/β-gal. This is in contrast to other studies which describe a short duration of expression (10 days or less) with recombinant adenoviral vectors administered to the airways or lung. However, when equivalent amounts of rAd/hIL-10 were delivered to the lung, an absence of neutralizing antibody responses was observed. These results are particularly unexpected in view of Ma, et al. (1998, J. Immunol. 161:1516-24) who reported a strong anti-vIL-10 antibody response to recombinant adenoviral vIL-10 vectors administered intravenously in DBA/I mice.

An additional set of experiments was performed to determine the presence of serum neutralizing anti-adenoviral antibodies in response to rAd/vIL-10 and rAd/hIL-10 administered to the lung. Serum was obtained from C57BL/6 mice 14 days following intratracheal administration of 1x10¹⁰ particles of rAd/IL-10 vectors. The presence of serum neutralizing antibodies was determined in accordance with the teaching of Example 8. The results are presented in Figure 7 of the attached drawings. Mice dosed with 1x10¹⁰ PN/mouse of rAd/β-gal by intratracheal route showed a strong serum neutralizing anti-adenoviral antibody response. In contrast, mice dosed with 1x10¹⁰ PN/mouse of rAd/hIL-10 and rAd/vIL-10 demonstrated a marked reduction in anti-adenoviral serum neutralizing antibodies.

This reduction in anti-adenoviral serum neutralizing antibodies suggests the ability of viral and human IL-10 to suppress anti-adenoviral humoral responses with rAd/IL-10 and facilitate readministration of rAd/IL-10 vectors or the co-administration of recombinant adenoviral vectors encoding different transgenes. In order to demonstrate this, rAd/vIL-10 and rAd/hIL-10 was co-administered with rAd/β-gal or with empty cassette. Briefly, 5x10⁹ particles of rAd/hIL-10 and rAd/vIL-10 was co-administered intratracheally with 5x10⁹ particles with rAd/β-gal or empty cassette vector. Levels of serum neutralizing anti-adenoviral antibodies were measured in accordance with the teaching of Example 8. The results are presented in Figure 8 of the attached drawings. As can be seen from the data presented, administration of rAd/vIL-10 with an empty cassette produced significantly fewer antibodies than administration of rAd/β-gal also with an empty cassette. These findings are in contrast to other studies with the intravenous administration of rAdIL-10 vectors which resulted in a strong neutralizing antibody response to rAd. These results indicate that local delivery of rAd/vIL-10 can suppress humoral responses to recombinant adenovirus vectors to a significant degree. Consequently, the practice of the present invention provides the potential to readminister viral vectors encoding vIL-10 or co-administer recombinant vectors encoding other transgenes.

In accordance with the data generated in this model system, the present invention provides a method of treating a mammalian organism suffering from a pulmonary inflammatory disease by the administration a recombinant expression vector comprising a viral IL-10 (vIL-10) expression cassette.

The term "mammalian organism" includes, but is not limited to, humans, pigs, horses, cattle, dogs, cats. The methods and compositions of the present invention may be used for the treatment of a variety of organisms suffering from of pulmonary inflammatory diseases. For example, the formulations and methods of the present invention may be used for the treatment of a variety of mammalian species suffering from such maladies including humans, pigs, horses, cattle, dogs, cats.

The term "pulmonary inflammatory disease" is used collectively to refer to those acute and chronic pathological conditions of the lung associated with inflammatory processes. Examples of acute pulmonary inflammatory diseases include ARDS, pneumonia, pneumonitis, lung infections, atelactasis, conditions associated with inflammatory lung injuries such as chemotherapeutic (e.g., bleomycin) induced lung injury, pancreatitis induced lung injury, hyperoxia induced lung injury, amiodarone induced pneumonitis, radiation pneumonitis, chlorine gas or smoke inhalation injuries, bronchiolitis obliterans/obstructive pneumonia (BOOP), viral and mycoplasmal pneumonias (e.g., Legionella and CMV lung), pneumoconioses. Examples of chronic pathological conditions of the lung include cystic fibrosis, silicosis, asbestosis, idiopathic pulmonary fibrosis (UIP), desquamative interstitial pneumonitis (DIP), hypersensitivity pneumonitis, interstitial pneumonitis, collagen vascular disease, sarcoidosis, coal worker's pneumoconiosis, bronchopulmonary dysplasia, inflammatory pseudotumor.

The term "recombinant expression vector" refers to viral and non-viral vectors prepared by conventional recombinant DNA techniques comprising a vIL10 expression cassette capable of expressing vIL-10 in a mammalian cell. A variety of viral and non-viral delivery vectors useful to achieve expression of nucleotide sequences in transduced cells are known in the art. See, e.g. Boulikas, T in Gene Therapy and Molecular Biology, Volume 1 (Boulikas, T. Ed.) 1998 Gene Therapy Press, Palo Alto, CA pages 1-172. A variety of vectors capable of expressing vEL-10 are also known in the art and may be used in the practice of the present invention (see, e.g. Moore, *et al*. United States Patent No. 5,736,390 issued April 7, 1998. Manickan, *et al*. (1998, Immunology 94(2):129-34), Denham, *et al*. (Ann Surg 1998 Jun;227(6):812-20), and Batteux, *et al*. (1999) Eur J Immunol 29(3):958-63 describe plasmid vectors for IL-10 gene therapy. Recombinant adenoviral vectors encoding human and viral IL-10 are described in Aparally, *et al*., Ma, *et al*. and Whalen, *et al*., Abstracts 764, 765, and 766 respectively, in Arthritis and Rheumatism (1997) Volume 40 Abstract Supplement: S 158. Additionally non-plasmid non-viral vectors containing a vIL-10 expression cassette may be employed for expression of vIL-10 in target tissues (Kundu, *et al*. (1998) Int J Cancer 76(5):713-9).

In one embodiment of the invention as exemplified herein, the vector is a viral vector. The terms virus(es) and viral vector(s) are used interchangeably herein. The viruses useful in the practice of the present invention include recombinantly modified enveloped or non-enveloped DNA and RNA viruses, preferably selected from baculoviridiae, parvoviridiae, picornoviridiae, herpesveridiae, poxviridae, adenoviridiae, or picornnaviridiae. The viral genomes may be modified by conventional recombinant DNA techniques to provide expression of vIL-10 and may be engineered to be replication deficient, conditionally replicating or replication competent. Chimeric viral vectors which exploit advantageous elements of each of the parent vector properties (See e.g., Feng, *et al*.(1997) Nature Biotechnology 15:866-870) may also be useful in the practice of the present invention. Minimal vector systems in which the viral backbone contains only the sequences needed for packaging of the viral vector and may optionally include a vIL-10 expression cassette may also be employed in the practice of the present invention. In some instances it may be advantageous to use vectors derived from different species from that to be treated which possess favorable pathogenic features such as avoidance of pre-existing immune response. For example, equine herpes virus vectors for human gene therapy are described in WO98/27216 published August 5, 1998. The vectors are described as useful for the treatment of humans as the equine virus is not pathogenic to humans. Similarly, ovine adenoviral vectors may be used in human gene therapy as they are claimed to avoid the antibodies against the human adenoviral vectors. Such vectors are described in WO 97/06826 published April 10, 1997.

Many viruses exhibit the ability to infect a broad range of cell types. However, in many applications one wishes to infect only a certain subpopulation of cells in an organism. Consequently, a variety of techniques have evolved to facilitate selective or "targeted" vectors to result in preferential infectivity of the mature viral particle of a particular cell type. Cell type specificity or cell type targeting may also be achieved in vectors derived from viruses having characteristically broad infectivities such as adenovirus by the modification of the viral envelope proteins. For example, cell targeting has been achieved with adenovirus vectors by selective modification of the viral genome knob and fiber coding sequences to achieve expression of modified knob and fiber domains having specific interaction with unique cell surface receptors. Examples of such modifications are described in Wickham, *et al*. (1997) J. Virol. 71(11):8221-8229 (incorporation of RGD peptides into adenoviral fiber proteins); Amberg, *et al*. (1997) Virology 227:239-244 (modification of adenoviral fiber genes to achieve tropism to the eye and genital tract); Harris and Lemoine (1996) TIG 12(10):400-405; Stevenson, *et al*. (1997) J. Virol. 71(6):4782-4790; Michael, *et al*.(1995) gene therapy 2:660-668 (incorporation of gastrin releasing peptide fragment into adenovirus fiber protein); and Ohno, *et al*. (1997) Nature Biotechnology 15:763-767 (incorporation of Protein A-IgG binding domain into Sindbis virus). Other methods of cell specific targeting have been achieved by the conjugation of antibodies or antibody fragments to the envelope proteins (see, e.g. Michael, *et al*. (1993) J. Biol. Chem. 268:6866-6869, Watkins, *et al*. (1997) Gene Therapy 4:1004-1012; Douglas, *et al*. (1996) Nature Biotechnology 14: 1574-1578. Viral vectors encompassing one or more of such targeting modifications may optionally be employed in the practice of the present invention to enhance the selective infection and expression of vIL-10 in lung tissues, especially epithelial tissues of the lung.

In one embodiment of the invention as exemplified herein, the vIL-10 vector is an adenoviral vector. The term adenoviral vector refers collectively to animal adenoviruses of the genus mastadenovirus including but no limited to human, bovine, ovine, equine, canine, porcine, murine and simian adenovirus subgenera. In particular, human adenoviruses includes the A-F sugenera as well as the individual serotypes thereof the individual serotypes and A-F subgenera including but not limited to human adenovirus types 1, 2, 3, 4, 4a, 5, 6, 7, 8, 9, 10, 11 (Ad11A and Ad 11P), 12,13,14,15,16,17,18,19,19a, 20, 21, 22, 23, 24, 25, 26, 27, 28, 29, 30, 31, 32, 33, 34, 34a, 35, 35p, 36, 37, 38, 39, 40, 41, 42, 43, 44, 45, 46, 47, 48, and 91. The term bovine adenoviruses includes but is not limited to bovine adenovirus types 1,2,3,4,7, and 10. The term canine adenoviruses includes but is not limited to canine types 1 (strains CLL, Glaxo, RI261, Utrect, Toronto 26-61) and 2. The term equine adenoviruses includes but is not limited to equine types 1 and 2. The term porcine adenoviruses includes but is not limited to porcine types 3 and 4. The use of adenoviral vectors for the delivery of exogenous transgenes are well known in the art. See e.g., Zhang, W-W. (1999) Cancer Gene Therapy 6:113-138. A particularly preferred embodiment of an adenoviral vector for expression of the vIL-10 sequence is a replication deficient human adenovirus of serotype 2 or 5 created by elimination of adenoviral E1 genes resulting in a virus which is substantially incapable of replicating in the target tissues.

The term "expression cassette" is used herein to define a nucleotide sequence capable of directing the transcription and translation of a vIL-10 coding sequence comprising regulatory elements operably linked to a vIL-10 coding sequence so as to result in the transcription and translation of a the vIL-10 sequence in a transduced mammalian cell.

The term "viral IL-10 coding sequence" refers to a nucleotide sequence encoding vIL-10. Viral IL-10 refers to IL-10 proteins derived from viral sources and functional analogs thereof. The term viral IL-10 refers to IL-10 molecules derived from viral sources such as the IL-10 molecule derived from Epstein-Barr Virus (EBV) as described in Moore, *et al*. United States Patent No. 5,736,390 issued April 7, 1998 the entire teaching of which is herein incorporated by reference. The term functional analogs refers to naturally occurring or synthetic peptidyl analogs of IL-10. It will be readily apparent to those of skill in the art that the sequences described may be used to isolate homologous vIL-10 molecules from viral genomes by techniques well known in the art and such homologous vIL-10 sequences may also be employed in the practice of the present invention. In the preferred practice of the invention, the vIL-10 molecule is derived from EBV.

The term "regulatory element" refers to promoters, enhancers, transcription terminators, polyadenylation sites, and the like. The term "operably linked" refers to a linkage of polynucleotide elements in a functional relationship. A nucleic acid sequence is "operably linked" when it is placed into a functional relationship with another nucleic acid sequence. For instance, a promoter or enhancer is operably linked to a coding sequence if it affects the transcription of the coding sequence. Operably linked means that the nucleotide sequences being linked are typically contiguous. However, as enhancers generally function when separated from the promoter by several kilobases and intronic sequences may be of variable lengths, some polynucleotide elements may be operably linked but not directly flanked and may even function in trans from a different allele or chromosome. The expression cassette may also contain other sequences aiding expression and/or secretion of the vIL-10 protein product.

The term "promoter" is used in its conventional sense to refer to a nucleotide sequence at which the initiation and rate of transcription of a coding sequence is controlled. The promoter contains the site at which RNA polymerase binds and also contains sites for the binding of regulatory factors (such as repressors or transcription factors). Promoters may be naturally occurring or synthetic. When the vector to be employed is a viral vector, the promoters may be endogenous to the virus or derived from other sources. The regulatory elements may be arranged so as to allow, enhance or facilitate expression of the transgene only in a particular cell type. For example, the expression cassette may be designed so that the transgene is under control of a promoter which is constituitively active, or temporally controlled (temporal promoters), activated in response to external stimuli (inducible), active in particular cell type or cell state (selective) constitutive promoters, temporal viral promoters or regulatable promoters.

The term "selective promoter" refers to promoters which are preferentially active in selected cell types and are commonly referred to as tissue specific promoters. A variety of tissue specific promoters are well known in the art. In the preferred practice of the invention the promoter is a promoter selectively active in epithelial, dendritic cells (e.g. CD11c) or macrophage cells (e.g. PU.1) or epithelial cells of the lung. Epithelial specific promoters are known in the art.

When the IL-10 vector is a replication competent viral vector, it is preferred to use a temporal promoter to achieve high levels of vIL-10 expression. The term "temporal promoters" refers to promoters which are transcriptionally active at a point later in the viral cycle. Examples of such temporally regulated promoters include the adenovirus major late promoter (MLP) and the herpes simplex virus Latent Activated Promoter.

The term "inducible promoter" refers to promoters which facilitate transcription of the vIL-10 sequence preferably (or solely) under certain conditions and/or in response to external chemical or other stimuli. Examples of inducible promoters are known in the scientific literature (See, e.g. Yoshida and Hamada (1997) Biochem. Biophys. Res. Comm. 230:426-430; Iida, *et al*. (1996) J. Virol. 70(9):6054-6059; Hwang, *et al*. (1997) J. Virol 71(9):7128-7131; Lee, *et al*. (1997) Mol. Cell. Biol. 17(9):5097-5105; and Dreher, *et al*. (1997) J. Biol. Chem. 272(46); 29364-29371. Examples of radiation inducible promoters are described in Manome, *et al*. (1998) Human Gene Therapy 9:1409-1417). In such manner, it is possible to regulate the expression of the vIL-10 in the target tissue by the administration of the inducer to organism.

In one embodiment of the invention, the method provided are applied to the treatment of acute pulmonary inflammatory diseases. One embodiment of the invention provides a method of treating a mammalian subject suffering from ARDS. As previously discussed, ARDS possesses a time course of increasing pathology. Consequently, early intervention is essential to maximize the therapeutic response and before the pathological cytokine cascades and sequeli are insurmountable. In the preferred practice of the invention, the recombinant IL-10 vector is administered to the lung no later than the third stage of the disease and most preferably in the first or second stages of the disease. Treatment at stage 4 should not yet be ruled out and the capacity to improve symptoms at such a late stage will have to be determined empirically or in a controlled experimental setting.

The determination of the optimal dosage regimen for treatment of the disease will be based on a variety of factors which are within the discretion of the attending health care provider, such as the progression of the disease at the time of treatment, age, weight, sex, etc. However, recombinant adenoviral vectors have been demonstrated to be safe and effective in the dosage range between 1x10⁵ and 1x10¹² viral particles per dose. Consequently administration of recombinant adenoviral vectors encoding vIL-10 may be used in such dosage ranges.

Surfactants are sometimes employed in the treatment of acute pulmonary inflammatory diseases. For example, surfactants are administered to the lungs of ARDS to prevent collapse of alveoli induced by limited or defective natural surfactants. It has also been observed that the surfactants also enhance the delivery of vectors across cell membranes, increasing the level of gene expression in the target cells. Consequently, in a preferred embodiment of the invention, vIL-10 gene therapy may be combined with this conventional treatment regimen resulting in enhanced combined efficacy in the treatment of acute pulmonary inflammatory diseases such as ARDS. The enhanced transduction may apply to the use of multiple delivery systems including recombinant viral vectors, DNA, DNA/lipid complexes, DNA/protein complexes and DNA/surfactant complexes. Examples of surfactants are sodium dodecyl sulfate (SDS) and lysolecithin, polysorbate 80, nonylphenoxypolyoxyethylene, lysophosphatidylcholine, polyethylenglycol 400, polysorbate 80, polyoxyethylene ethers, polyglycol ether surfactants and DMSO.

As previously demonstrated, the administration of recombinant vectors expression vIL-10 has been demonstrated to suppress the humoral immune response commonly associated with the delivery of gene therapy vectors. Consequently, the administration of recombinant vectors encoding vIL10 permits the repeat administration of such vectors or the co-administration of other vectors to therapeutic advantage without rapid clearance by the immune system. Consequently, the methods of the present invention may also be employed in the treatment of chronic pulmonary inflammatory diseases. Consequently, in one embodiment of the invention, therapeutically effective amount of a recombinant vector encoding vIL-10 is administered to an organism suffering from a chronic pulmonary inflammatory disease. The vector is preferably administered directly to the lung tissues over the duration of the disease which may be for many years in an human subject. In instances where the vector is a replication deficient adenoviral vector, the preferred dosage regimen is to administer from 1x10⁹ to 1x10¹³ viral particles to the lung in a primary dose. Follow-on dosages will vary with the frequency of treatment and the severity of the disease, but in general will be lower by approximately 1 to 2 logs if the vIL-10 vector is provided approximately every 21 days. An increased frequency will necessitate a lower dose as will a milder etiology. The exact parameters for administration to a mammalian subject may be determined in accordance with dose response evaluation of the individual patient.

Furthermore, the immunosuppressive effects of vIL-10 vectors enable the foregoing treatment regimen to be practiced in combination with other treatment regimens conventionally employed in the treatment of pulmonary inflammatory diseases such as CFTR and α-1 antitrypsin. In particular a recombinant vIL-10 expression vector may be co-administered with an recombinant expression vector expressing a second therapeutic gene useful in the treatment of pulmonary inflammatory diseases such as CFTR and α-1 antitrypsin, secretory leukocyte protease inhibitor, superoxide dismutase, catalase, prostaglandin synthase, and tissue inhibitors of metalloproteinase (TIMP). The term "co-administration" refers to the administration within a sufficient time such that the therapeutic effect of one element has not been eliminated prior to the administration of the second agent. The vIL-10 vector may be administered before, with or following administration of the second vector encoding the additional therapeutic agent. Most preferably, the vIL-10 vector and the expression vector expressing a second therapeutic gene are administered at approximately the same time.

In another embodiment of the invention, a recombinant expression vector may be constructed so as to permit delivery of both the vIL-10 and an additional therapeutic gene,on a single vector. For example, a recombinant vector containing two expression cassettes, one expressing vIL-10 and the other expressing CFTR could be provided so as to permit essentially simultaneous delivery of these agents to the lung.

As previously indicated, the administration of vIL-10 vectors significantly reduces the humoral immune response to recombinant vectors, particularly adenoviral vectors. However, in order to more fully suppress this immune response, the above treatment regimens may also be supplemented by the co-administration of conventional immunosuppressive agents. Examples of immunosuppressive agents include cyclosporine, azathioprine, methotrexate, cyclophosphamide, lymphocyte immune globulin, antibodies against the CD3 complex, adrenocorticosteroids, sulfasalzaine, FK-506, methoxsalen, and thalidomide.

The recombinant vIL-10 vectors are formulated for administration to a mammalian subject in accordance with procedures and compounds well known in the art. When the vector used to deliver the vIL-10 gene is a non-viral vector, a variety of means well known in the art may be used to formulate such vectors for administration to a mammalian subject. In one embodiment, the vIL-10 vectors may be encapsulated in liposomes. The term "liposomes" includes emulsions, foams, micelles, insoluble monolayers, liquid crystals, phospholipid dispersions, lamellar layers and the like. The delivery of nucleotide sequences to target cells using liposome carriers is well known in the art. A variety of methods are available for preparing liposomes, as described in, e.g., Szoka *et al*. Ann. Rev. Biophys. Bioeng. 9:467 (1980), Szoka, *et al*. United States Patent No 4,394,448 issued July 19, 1983, as well as U.S. Patent Nos. 4,235,871, 4,501,728, 4,837,028, and 5,019,369, incorporated herein by reference. Liposomes useful in the practice of the present invention may be formed from one or more standard vesicle-forming lipids, which generally include neutral and negatively charged phospholipids and a sterol, such as cholesterol. Examples of such vesicle forming lipids include DC-chol, DOGS, DOTMA, DOPE, DOSPA, DMRIE, DOPC, DOTAP, DORIE, DMRIE-HP, n-spermidine cholesterol carbamate and other cationic lipids as disclosed in United States Patent No. 5,650,096. The selection of lipids is generally guided by consideration of, e.g., liposome size, acid lability and stability of the liposomes in the blood stream. Additional components may be added to the liposome formulation to increase serum half-life such as polyethylene glycol coating (so called "PEG-ylation") as described in United States Patent Nos. 5,013,556 issued May 7, 1991 and 5,213,804 issued May 25, 1993. In order to ensure efficient delivery of the therapeutic gene to a particular tissue or organ, it may be advantageous to incoporate elements into the non-viral delivery system which facilitate cellular targeting. For example, a lipid enccpulated vector may incorporate modified surface cell receptor ligands to facilitate targeting. Examples of such ligands includes antibodies, monoclonal antibodies, humanized antibodies, single chain antibodies, chimeric antibodies or functional fragments (Fv, Fab, Fab') thereof. Alternatively, the DNA constructs of the invention can be linked through a polylysine moiety to a targeting moiety as described in Wu, *et al*. United States Patent No. 5,166,320 issued November 24, 1992 and Wu, *et al*., United States Patent No. 5,635,383 issued June 3, 1997, the entire teachings of which are herein incorporated by reference. The non-viral vIL-10 vectors may also be formulated for oral formulation in complex with chitosan and delivered by the use of porous nanoparticles. (Roy, *et al*. (1999) Nature Medicine 5:387-391. Examples of formulations for the delivery of transgenes via non-viral delivery systems to the airway epithelia is described in Debs and Zhu, United States Patent No 5,641,622 issued June 24, 1997.

In a preferred embodiment of the invention, particularly when the vIL-10 vector is a viral vector, the vector is delivered in combination or complexed with a delivery enhancing agent to increase uptake of the viral particles across the lung epithelial surface. The terms "delivery enhancers" or "delivery enhancing agents" are used interchangeably herein and includes agents which facilitate the transfer of the nucleic acid or protein molecule to the target cell. Examples of such delivery enhancing agents detergents, alcohols, glycols, surfactants, bile salts, heparin antagonists, cyclooxygenase inhibitors, hypertonic salt solutions, and acetates. Alcohols include for example the aliphatic alcohols such as ethanol, N-propanol, isopropanol, butyl alcohol, acetyl alcohol. Glycols include glycerine, propyleneglycol, polyethyleneglycol and other low molecular weight glycols such as glycerol and thioglycerol. Acetates such as acetic acid, gluconic acid, and sodium acetate are further examples of delivery-enhancing agents. Hypertonic salt solutions like 1M NaCl are also examples of delivery-enhancing agents. Bile salts such as taurocholate, sodium taurodeoxycholate, deoxycholate, chenodesoxycholate, glycocholic acid, glycochenodeoxycholic acid and other astringents such as silver nitrate may be used. Heparin-antagonists like quaternary amines such as protamine sulfate may also be used. Anionic, cationic, zwitterionic, and nonionic detergents may also be employed to enhance gene transfer. Exemplary detergents include but are not limited to taurocholate, deoxycholate, taurodeoxycholate, cetylpyridium, benalkonium chloride, Zwittergent 3-14 detergent, CHAPS (3-[(3-Cholamidopropyl) dimethylammoniol]-1-propanesulfonate hydrate), Big CHAP, Deoxy Big CHAP, Triton-X-100 detergent, C12E8, Octyl-B-D-Glucopyranoside, PLURONIC- F68 detergent, Tween 20 detergent, and TWEEN 80 detergent (CalBiochem Biochemicals).

The formulations may also include carrier molecules conventionally used in the formulation of pharmaceutical agents. The term "carriers" refers to compounds commonly used on the formulation of pharmaceutical compounds used to enhance stability, sterility and deliverability of the therapeutic compound. When the viral, non-viral or protein delivery system is formulated as a solution or suspension, the delivery system is in an acceptable carrier, preferably an aqueous carrier. A variety of aqueous carriers may be used, e.g., water, buffered water, 0.8% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

*In vivo* administration will typically employ the compositions of the present invention formulated for direct administration to the lung such as coated microparticles, aerosolized and microparticle encapsulated formulations and the like.

### Examples

The following examples provide the methodology and results of experiments demonstrating the construction of exemplary hybrid vectors of the invention. It will be apparent to those of skill in the art to which the invention pertains, the present invention may be embodied in forms other than those specifically disclosed in these examples, without departing from the spirit or essential characteristics of the invention. The particular embodiments of the invention described below, are therefore to be considered as illustrative and not restrictive. In the following examples, "g" means grams, "ml" means milliliters, "°C" means degrees Centigrade, "min." means minutes, "FBS" means fetal bovine serum.

### Example 1. Construction of A Recombinant Adenovirus Expressing Human IL-10

A derivative of human adenovirus serotype 5 (described in Smith, et al., (1997) Circulation 96:1899-1905) was used as the source of viral DNA backbone. It is deleted in early region 1, polypeptide IX, early region 3 and part of early region 4. Specifically, the vector contains a deletion of base pairs 355 to 3325 to eliminate E1a and E1b functions, a deletion of base pairs 3325 to 4021 to eliminate protein IX function and a deletion of base pairs 28592 to 30470 to eliminate E3 functions. See Wills, *et al*. (1994) Human Gene Therapy 5:1079-1088.

Recombinant adenoviruses were constructed using standard homologous recombination methods in substantial accordance with the teaching of Graham and Prevec (1991) Meth. Mol. Biol. 7:109-128 . A recombinant transfer plasmid (pACN) was constructed to aid in the transfer of gene expression cassettes into viral vectors. Plasmid pACN is based on plasmid pBR322 and contains, in order, the adenovirus 5 inverted terminal repeat and packaging signal (Ad5 base pairs 1-358), the human cytomegalovirus immediate early enhanver/promoter (CMV), the adenovirus type 2 tripartite leader sequence (TPL), a multiple cloning site, and a DNA sequence equivalent to base pairs 4021 through 10457 of human adenovirus serotype 2. The preparation of pACN is described in Wills, et al. supra.

In order to generate a recombinant adenoviral vector expressing hIL-10, a cDNA sequence encoding IL-10 was isolated from the pDSRG-IL10 plasmid (obtained from DNAX Research Institute, Inc., Palo Alto, CA). The pDSRG-IL10 vector was digested with HindIII and EcoR1 restriction endonucleases and the fragment containing the hIL-10 coding sequence was isolated by gel electrophoresis. The pBK-RSV plasmid (commercially available from Stratagene, 11011 North Torrey Pines Road, La Jolla, CA 92037) was linearized by digestion with HindIII and EcoR1. This linearized fragment was ligated with the HindIII-EcoR1 fragment containing the hIL-10 gene and annealed to produce vector pBK-RSV-IL10. Vector pBK-RSV-IL10 was then digested with XbaI and BamHI and a 592 base pair XbaI-BamHI fragment containing the hIL-10 sequence was isolated by gel electrophoresis. Plasmid pACN (prepared in accordance with the teaching of Example 1) was digested with the XbaI and BamHI and ligated with the 592 base pair XbaI-BamHI containing the hIL-10 sequence to generate transfer plasmid pACN-hIL10.

pACN-hIL10 was linearized with restriction endonuclease NgoM1 and co-transfected into 293 cells with the large Cla1 digested fragment of a modified dl327 adenoviral vector. (Wills, et al. (1994) supra) using a MBS Mammalian Transfection Kit calcium phosphate kit (Stratagene). The human embryonic kidney cell line 293 capable of supplying adenoviral E1 functions in trans (ATCC CRL-1573 ) used for the propagation of recombinant adenoviruses, was grown in Dulbecco's modified Eagle's medium containing 10% bovine calf serum. The co-transfection results in recombination events between the transfer vector and the Cla1 fragment resulting in production of a recombinant adenovirus vectors expressing hIL-10. Recombinants were isolated by placque assay from the transfected 293 cells and further placque purified by two rounds placque purification. Viral plaques were isolated and purified by column chromatography (Huyghe, et al., (1995) Hum. Gene Ther. 6:1403-1416). These viruses were characterized by both restriction enzyme digestion and DNA sequencing across the transgene and the promoter sequences.

### Example 2. Construction of A Recombinant Adenovirus Expressing Viral IL-10

The construction of a recombinant adenovirus expressing vIL-10 was performed in substantial accordance with the teaching of Example 1 above except that the pACN-BCRF1 was produced by the following method. Plasmid pBCRF1(SR-α) containing the cDNA encoding vIL-10 was obtained from DNAX Research Institute. An E.coli cell culture (MC1061) containing this vector has been deposited with the American Type Culture Collection as accession number 68193 and is described in United States Patent No. 5,736,390. Plasmid pBCRF1(SR-α) was modified by PCR to include Xba1 and BamHI restriction on the 5' and 3' ends of the vIL-10 cDNA sequence, respectively. The PCR primers used were 5' Xba1 containing sequence 5'-TGCTCTAGAGCTCCATCATGGAGCGAAGGTTAGTG-3' and the 3' antisense BamHI containing sequence is 5'-CGCGGATCCGCGTCACCTGGCTTTAATTGT-3'. The resulting 530 bp fragment was subcloned into the XbaI-BamHI digested pACN transfer plasmid and ligated.

### Example 3. Construction of Empty Cassette Virus

A control recombinant adenovirus was prepared in substantial accordance with the teaching of Example 1 above except that the transfer plasmid pACN contained no cDNA protein coding sequence.

### Example 4. Administration of Vectors in a Mouse Lung Inflammation Model

Specific pathogen-free male and female C57BL/6 mice were either obtained from The Jackson Laboratory (Bar Harbor, ME) (20-25 gm) or from the University of Florida Health Science Center Animal Resources Department. Mice were housed in a barrier facility in groups of five per cage for the duration of the experiment. Mice were anesthetized with 35 mg/kg body weight of intra-peritoneal sodium pentobarbital. After induction of anesthesia, a mid-line incision was made in the neck, the fat pad was elevated caudally, and the trachea was visualized. Under direct visualization, the trachea was canulated with a sterile 30 gauge needle and 32 microliters of buffer or adenoviral vector encoding β-gal (rAd/β-gal), hIL-10 (rAd/hEL-10), vIL-10 (rAd/vIL-10) and an empty cassette control virus (rAd/empty) prepared in substantial accordance with the teaching of Examples 1-3 herein were administered at a dose of 1 x 10¹⁰ viral particles per animal. The incision was then closed and the mice were returned to their cage to recover.

Mice were sacrificed by cervical dislocation at one, three, five, seven, nine or fourteen days following viral instillation (hereinafter referred to as day 1, 3, 5, 7, 9 or 14). Blood was collected via a retro-orbital venipuncture utilizing a heparinized capillary tube. The blood was centrifuged for 10 minutes at 10,000 rpm and the serum was collected. The lungs and trachea were removed en bloc and snap frozen in liquid nitrogen.

### Example 5. Measurement of β-Galactosidase Expression In the Lung

The snap frozen lungs prepared in accordance with the teaching of Example 4 were homogenized for one minute in 15 weight:volumes of phosphate-buffered saline. The homogenized sample was centrifuged for 20 minutes at 10,000 rpm at 4°C, and the supernatant collected. β-galactosidase activity was detected in the lung using the GalactoLight™ chemiluminescent reporter gene assay system (commercially available from Tropix Inc., 47 Wiggins Avenue, Bedford, MA 01730) in substantial accordance with the instructions provided by the manufacturer. The reaction was carried out for 1 hour at room temperature and the b-galactosidase activity was measured using a Monolight 500 luminometer (obtained from EG&G Wallac Inc., Gaithersburg, MD). Baseline β-galactosidase activity was obtained from the lung of an untreated animals analyzed simultaneously, and was subtracted from the β-galactosidase measurements obtained from treated mice. Baseline β-galactosidase activity from the lungs of control animals was less than 1% of peak activity seen in lungs from mice instilled with adenoviral vectors delivering β-galactosidase cDNA.

### Example 6. Measurement of Cytokines in Plasma and Lung Homogenates

Bioactive TNF-α was measured in plasma and lung homogenates using the TNF-α sensitive WEHI 164 clone 13 murine fibrosarcoma cell line in substantial accordance with the teaching of Espevik & Nissen-Meyer (1986) J. Immunol. Methods 95:99-105. Briefly, the cells were cultured in 96 well plates in complete medium with 1 µg/ml actinomycin D, and then exposed to either lung homogenate (prepared in accordance with the teaching of Example 4 above), serum samples diluted one to five, or increasing concentrations of human TNF-α. Cellular viability was determined by the reduction of 3-[4,5-dimethylthiazol-2-yl]-2,5-diphenyltetrazolium bromide and quantitated spectrophotometrically at an absorbance at 570 nm. A standard curve was generated with human TNF-α, and the sensitivity of the assay was 5-25 pg/mL.

Murine IL-6, murine IL-1 and human and viral IL-10 levels in the lung homogenates, and in the plasma where appropriate were measured by sandwich ELISA. Murine IL-6 and human IL-10 were measured using a mouse IL-6 ELISA kit and a human IL-10 ELISA kit (commercially available from Endogen, Inc. 30 Commerce Way, Woburn, Ma as catalog numbers EM-IL6 and EH-IL10 respectively) in substantial accordance with the manufacturer's instructions. Murine IL-1 was measured using a murine IL-1 ELISA kit (commercially available from R &D Systems, 614 McKinley Place NE, Minneapolis, MN 55413 as Catalog No. M1000) in substantial accordance with the manufacturer's instructions

Pulmonary neutrophil sequestration in the lungs was quantitated by measuring tissue myeloperoxidase (MPO) content in accordance with the following procedure. Snap frozen lungs (-70°C) were weighed and homogenized for one minute in 15 weight:volumes of 0.01 M KH₂PO₄ with 1 mM EDTA (PE Buffer). Following homogenization, the resultant pellet was resuspended in 13.7 mM cetyltrimethylammonium bromide (C-TAB) buffer with 50 mM acetic acid, using the same volume of C-TAB as PE buffer. The resuspended pellet was then sonicated for forty seconds at setting 60% on the using a Sonic Dismembrator, Model 300 sonicator (available from Fischer Scientific), and centrifuged at 10,000 rpm for 15 minutes. The resultant supernatant was collected and incubated in a water bath for 2 hours at 60°C. MPO activity was then measured in this solution by H₂O₂-dependent oxidation of 3.3'5.5'-tetramethyl-benzidine, which generates a colorimetric reaction. Spectrophotometric absorbance was determined at 650 nm and compared with a linear standard curve with a sensitivity of 0.03125 EU.

### Example 7. Determination of Humoral Immune Response

To determine whether a humoral response develops against the expressed b-galactosidase or human IL-10, a direct ELISA for antibodies (IgG or IgM) was performed in substantial accordance with the teaching of Solorzano, Kaibara, et al. (1998) Journal of Applied Physiology 84:1119-1130. Briefly, recombinant β-galactosidase (obtained from Sigma Chemicals Inc., St. Louis, Mo.) or human IL-10 (obtained from Schering-Plough Research Institute, Kenilworth, NJ.) were coated onto 96 well Coming flat-bottomed, polystyrene ELISA plates. After blocking the plates with Tris-buffered saline (TBS) containing 5% bovine serum albumin (BSA Sigma Fraction V) and 1% fat-free dry milk (Alba), mouse serum obtained at baseline and at 14 days after intratracheal instillation were diluted from 1:50 through 1:100,000 in TBS-5% BSA and applied to the wells (100 µl). The plates were washed and 1:3000 diluted horseradish peroxidase-conjugated, goat, antimouse IgG or antimouse IgM immunoglobulin (commercially available from Promega, 2800 Woods Hollow Road, Madison WI 53711) was added. Results were visualized with 3,3', 5,5' tetramethylbenzidine. A positive antibody response was recorded when the absorbance from samples at day 14 were at least two fold greater than the maximal absorbance determined from samples at day zero. The quantities of antibody were estimated from the highest inverse dilution of the sample producing a positive signal.

### Example 8. Measurement of Serum Neutralizing Antibody Response

A functional assay to determine the neutralizing capacity of antisera to prevent the capacity of recombinant adenoviral vectors to infect and transduce HeLa cells was performed in accordance with the following procedure. Antisera were diluted from 1:20 to 1:2560 by a 1:2 serial dilution. The diluted antisera were mixed with 8 x 10⁸ particles per milliliter of a recombinant adenoviral vector expressing the green fluorescent protein (rAd/GFP) at a 1:1 ratio with serially diluted antisera samples and incubated for one hour at 37 degrees C. The mixture was then placed on HeLa cells in a 96 well plate and incubated overnight to permit transduction. The plates were then measured for fluorescence intensity at 450 nm using a CytoFluor Multi Reader Series 4000 spectrophotometric plate reader (commercially available from Perseptive Biosystems, Inc.). The resulting fluorescence units were normalized against untreated HeLa cells containing only media and compared with maximal fluorescence (HeLa cells transduced with 4x10⁸ particles per milliliter rAd/GFP). The neutralizing antibody titer was assessed by determining the reverse titer at 50% absorption of normalized maximal absorption.

## Claims

1. The use of a recombinant expression vector comprising a viral IL-10 (vIL-10) expression cassette for the preparation of a medicament for the treatment of a mammalian organism suffering from a pulmonary inflammatory disease, wherein said medicament is suitable to be administered to the lung.

2. The use of claim 1, wherein the vector is a viral vector.

3. The use of claim 2, wherein the vector is an adenoviral vector.

4. The use of claim 3, wherein the adenoviral vector is replication deficient.

5. The use of claim 1, wherein the recombinant expression vector is a non-viral vector.

6. The use of claim 5, wherein the non-viral vector is a plasmid.

7. The use of claim 4 or 6, wherein the expression cassette comprises a constituitively active promoter.

8. The use of claim 5 or 7, wherein the vIL-10 coding sequence is the vIL-10 cDNA sequence derived from EBV.

9. The use of claim 5, wherein the recombinant expression vector is further complexed with a surfactant.

10. The use of claim 1, wherein said medicament is for the co-administration of a recombinant expression vector capable of directing the expression of CFTR and 1 α-antitrypsin, secretory leukocyte protease inhibitor, superoxide dismutase, catalase, prostaglandin synthase, and tissue inhibitors of metalloproteinase (TIMP).

11. The use of claim 1, wherein said recombinant expression vector further comprises an expression cassette capable of directing the expression of CFTR and α-antitrypsin, secretory leukocyte protease inhibitor, superoxide dismutase, catalase, prostaglandin synthase, and tissue inhibitors of metalloproteinase (TIMP).

12. The use of claim 3, wherein the pulmonary imflammatory disease is an acute pulmonary inflammatory disease.

13. The use of claim 12, wherein said acute pulmonary inflammatory disease is ARDS.

14. The use of claim 13, wherein the vector is suitable to be administered in the first or second stages of ARDS.

15. The use of claim 14, wherein the adenoviral vector is suitable to be administered at a dosage of from 1 x 10⁸ to 1 x 10¹² particles.

## Patentansprüche

1. Verwendung eines rekombinanten Expressionsvektors, der eine virale IL-10 (vIL-10)-Expressionskassette umfaßt, zur Herstellung eines Medikamentes zur Behandlung eines Säugetier-Organismus, der an einer pulmonalen Entzündungserkrankung leidet, wobei das Medikament geeignet ist, an die Lunge verabreicht zu werden.

2. Verwendung nach Anspruch 1, **dadurch gekennzeichnet, daß** der Vektor ein viraler Vektor ist.

3. Verwendung nach Anspruch 2, **dadurch gekennzeichnet, daß** der Vektor ein adenoviraler Vektor ist.

4. Verwendung nach Anspruch 3, **dadurch gekennzeichnet, daß** der adenovirale Vektor replikationsdefizient ist.

5. Verwendung nach Anspruch 1, **dadurch gekennzeichnet, daß** der rekombinante Expressionsvektor ein nicht-viraler Vektor ist.

6. Verwendung nach Anspruch 5, **dadurch gekennzeichnet, daß** der nicht-virale Vektor ein Plasmid ist.

7. Verwendung nach Anspruch 4 oder 6, **dadurch gekennzeichnet, daß** die Expressionskassette einen konstitutiv aktiven Promotor umfaßt.

8. Verwendung nach Anspruch 5 oder 7, **dadurch gekennzeichnet, daß** die für vIL-10 kodierende Sequenz die von EBV abgeleitete vIL-10 cDNA-Sequenz ist.

9. Verwendung nach Anspruch 5, **dadurch gekennzeichnet, daß** der rekombinante Expressionsvektor ferner mit einer Oberflächen-aktiven Substanz komplexiert ist.

10. Verwendung nach Anspruch 1, **dadurch gekennzeichnet, daß** das Medikament für die Co-Verabreichung eines rekombinanten Expressionsvektors ist, welcher in der Lage ist, die Expression von CFTR und 1 α-Antitrypsin, sekretorischem Leukocyten-Protease-Inhibitor, Superoxid-Dismutase, Katalase, Prostaglandin-Synthase und Gewebe-Inhibitoren der Metalloproteinase (TIMP) zu steuern.

11. Verwendung nach Anspruch 1, **dadurch gekennzeichnet, daß** der rekombinante Expressionsvektor ferner eine Expressionskassette umfaßt, die in der Lage ist, die Expression von CFTR und α-Antitrypsin, sekretorischem Leukozyten-Protease-Inhibitor, Superoxid-Dismutase, Katalase, Prostaglandin-Synthase und Gewebe-Inhibitoren der Metalloproteinase (TIMP) zu steuern.

12. Verwendung nach Anspruch 3, **dadurch gekennzeichnet, daß** die pulmonale Entzündungserkrankung eine akute pulmonale Entzündungserkrankung ist.

13. Verwendung nach Anspruch 12, **dadurch gekennzeichnet, daß** die aktue pulmonale Entzündungserkrankung ARDS ist.

14. Verwendung nach Anspruch 13, **dadurch gekennzeichnet, daß** der Vektor geeignet ist, während der ersten oder zweiten Stufe von ARDS verabreicht zu werden.

15. Verwendung nach Anspruch 14, **dadurch gekennzeichnet, daß** der adenovirale Vektor geeignet ist, in einer Dosierung von 1 x 10⁸ bis 1 x 10¹² Partikeln verabreicht zu werden.

## Revendications

1. Utilisation d'un vecteur d'expression recombinant comprenant une cassette d'expression de IL-10 virale (vIL-10) en vue de la préparation d'un médicament destiné au traitement d'un organisme mammalien souffrant d'une maladie inflammatoire pulmonaire, dans laquelle ledit médicament est adapté à une administration dans le poumon.

2. Utilisation selon la revendication 1, dans laquelle le vecteur est un vecteur viral.

3. Utilisation selon la revendication 2, dans laquelle le vecteur est un vecteur adénoviral.

4. Utilisation selon la revendication 3, dans laquelle le vecteur adénoviral est défectif pour la réplication.

5. Utilisation selon la revendication 1, dans laquelle le vecteur d'expression recombinant est un vecteur non viral.

6. Utilisation selon la revendication 5, dans laquelle le vecteur non viral est un plasmide.

7. Utilisation selon la revendication 4 ou 6, dans laquelle la cassette d'expression comprend un promoteur constitutivement actif.

8. Utilisation selon la revendication 5 ou 7, dans laquelle la séquence codant pour la cassette vIL-10 est la séquence d'ADNc de la cassette vIL-10 de l'EBV.

9. Utilisation selon la revendication 5, dans laquelle le vecteur d'expression recombinant est en outre complexé avec un agent tensioactif.

10. Utilisation selon la revendication 1, dans laquelle ledit médicament est destiné à être co-administré avec un vecteur d'expression recombinant capable de diriger l'expression du régulateur de la perméabilité transmembranaire de la mucoviscidose (Cystic Fibrosis Transmembrane Conductance Regulator, CFTR), et de la α-1 antitrypsine, d'un inhibiteur de la protéase de leucocyte sécrétoire, de la superoxyde dismutase, d'une catalase, d'une prostaglandine synthétase, et d'inhibiteurs tissulaires des métalloprotéases (TIMP).

11. Utilisation selon la revendication 1, dans laquelle ledit vecteur d'expression recombinant comprend en outre une cassette d'expression capable de diriger l'expression du CFTR, et de la α-1 antitrypsine, d'un inhibiteur de la protéase de leucocyte sécrétoire, de la superoxyde dismutase, d'une catalase, d'une prostaglandine synthétase, et d'inhibiteurs tissulaires des métalloprotéases (TIMP).

12. Utilisation selon la revendication 3, dans laquelle la maladie inflammatoire pulmonaire est une maladie inflammatoire aiguë.

13. Utilisation selon la revendication 12, dans laquelle ladite maladie inflammatoire pulmonaire aiguë est le syndrome de détresse respiratoire aiguë de l'adulte (ARDS).

14. Utilisation selon la revendication 13, dans laquelle le vecteur est adapté à une administration dans les premier et second stades de l'ARDS.

15. Utilisation selon la revendication 14 dans laquelle le vecteur adénoviral est adapté à une administration à une dose de 1 x 10⁸ à 1 x 10¹² particules.
